# FASCICULE DE BREVET EUROPEEN

(11) **EP 3 621 563 B1**
(45) Date de publication et mention de la délivrance du brevet: **24.03.2021**
(21) Numéro de dépôt: 18728445.0
(22) Date de dépôt: 11.05.2018
(51) Int. Cl.: A61F 5/01

(54) **ORTHÈSE DE MAIN**
HANDORTHESE
HAND ORTHOSIS

(30) Priorité: 12.05.2017 FR 1754220
(43) Date de publication de la demande: 18.03.2020
(73) Titulaire: Cizeta Medicali France, 18200 Saint-Amand-Montrond (FR)
(72) Inventeur: DUBOURG, Charles, 18200 Saint Amand Montrond (FR)
(74) Mandataire: Gauchet, Fabien Roland
(86) Numéro de dépôt international: PCT/FR2018/051163
(87) Numéro de publication internationale: WO 2018/206903

(56) Documents cités:
- EP-A1- 0 809 988
- FR-A1- 2 857 854
- FR-A1- 2 876 901
- FR-A1- 2 990 344
- US-A- 4 407 499
- US-A1- 2004 143 205
- US-A1- 2004 176 714
- US-B2- 8 246 560

## Description

### DOMAINE TECHNIQUE DE L'INVENTION

L'invention se rapporte au domaine des orthèses de main aptes à immobiliser plusieurs os métacarpiens, plus spécifiquement à immobiliser deux métacarpiens jointifs et plus concrètement destinées à immobiliser l'articulation métacarpo-phalangienne.

Autrement dit, la présente invention concerne une orthèse destinée à être placée sur une main d'un utilisateur, notamment pour le traitement de la fracture des métacarpiens de la main.

### ETAT DE LA TECHNIQUE ANTERIEURE

Il existe une grande variété de fractures de métacarpiens, elles peuvent être articulaires ou extra-articulaires. Les fractures extra-articulaires peuvent tolérer de légers déplacements sans toutefois nécessiter un traitement chirurgical. Les fractures articulaires et les fractures extra-articulaires déplacées feront l'objet de traitements chirurgicaux le plus souvent par vis ou par plaques. Le traitement de la fracture de métacarpien par la voie orthopédique vise d'une part à diminuer la douleur et d'autre part à immobiliser la zone de fracture pour favoriser la cicatrisation.

Il existe déjà plusieurs types d'immobilisation de la main utilisées pour le traitement de la fracture du métacarpien.

Selon une première réalisation connue, une telle orthèse consiste à réaliser un moulage en résine. L'intervention d'un médecin est généralement requise pour réaliser l'immobilisation. De plus, la résine ne peut être facilement adaptée aux conditions de port.

Selon une deuxième réalisation connue, une telle orthèse consiste en une sangle textile, éventuellement pourvue de raidisseurs, que l'utilisateur vient adapter et fixer autour du doigt et de la main. Cette orthèse est généralement difficile à mettre en place par l'utilisateur seul. De plus un mauvais positionnement peut nuire au traitement orthopédique.

Selon une troisième réalisation connue, une telle orthèse consiste en un moulage en un matériau thermoformable. L'intervention d'un orthésiste est généralement requise, pour réaliser cette orthèse sur mesure. Une orthèse de ce type s'avère donc contraignante et couteuse à réaliser. De plus elle ne peut être facilement adaptée aux conditions de port.

Selon une quatrième réalisation connue, une telle orthèse de série consiste à immobiliser le poignet, la main et le doigt en rectitude. Cette orthèse est généralement difficile à mettre en place par l'utilisateur seul. De plus un mauvais positionnement peut nuire au traitement orthopédique.

Selon une cinquième réalisation connue, une telle orthèse consiste à immobiliser le poignet, la main en position intrinsèque en mettant les doigts en rectitude. Cette orthèse est généralement non fonctionnelle car les doigts sont emprisonnés ce qui ne permet pas la préhension.

Selon encore une autre forme de réalisation, l'orthèse consiste à immobiliser le poignet, la main en position intrinsèque. Cette orthèse ne permet pas de s'adapter à toutes les variantes d'atteintes des métacarpiens. Cette orthèse est généralement difficile à adapter aux variétés d'atteintes des métacarpiens

Dans le document US 8 246 560 on décrit un système qui permet l'immobilisation du quatrième et du cinquième métacarpes, grâce à une orthèse spécifique. Cette orthèse ne permet en fait qu'une immobilisation entre ces deux métacarpes ; elle est donc très spécifique. De plus les deux doigts sont ici immobilisés en contact l'un avec l'autre ; on ne prévoit pas d'écartement entre les doigts, ce qui peut être ennuyeux car l'articulation est alors totalement immobilisée.

Les mêmes remarques et fonctions s'appliquent pour le brevet US 6 953 441 qui concerne un système pour immobiliser les 4^{ème} et 5^{ème} doigts de la main ainsi que le poignet en position d'extension. Ce système comprend une pièce rigide associée à des bandeaux de maintien en position.

Le brevet US 7 442 177 illustre un système non amovible qui permet uniquement l'immobilisation des 4^{ème} et 5^{ème} métacarpiens, depuis le poignet.

On connait encore la demande internationale WO 2013006178 qui montre un système dynamique pour l'extension et la flexion du poignet et des doigts de la main.

On constate que les systèmes connus sont dédiés à l'immobilisation de deux doigts de la main généralement les 4^{ème} et 5^{ème} métacarpiens ; ils permettent uniquement cette immobilisation, ce qui est assez restrictif.

On peut en effet avoir besoin d'immobiliser d'autres doigts ; les systèmes proposés ne sont pas bien adaptés à ces cas de figure ; des adaptations sont alors nécessaires à partir des systèmes existants, ce qui n'est pas totalement satisfaisant dans la mesure où ces systèmes ne sont pas initialement conçus pour immobiliser d'autres doigts.

Par ailleurs les systèmes connus prévoient un appui pour l'immobilisation : soit dorsal soit palmaire. Ceci n'est pas non plus satisfaisant car on autorise ainsi un léger mouvement là où aucun appui n'est prévu.

Les orthèses connues ne prévoient pas davantage un léger écartement entre les deux doigts immobilisés ; or on sait qu'un tel écartement est bénéfique car il permet une meilleure immobilisation.

### EXPOSE DE L'INVENTION

L'invention vise à remédier aux inconvénients de l'état de la technique et notamment à prévoir un système d'immobilisation ou orthèse apte à immobiliser deux articulations métacarpo-phalangienne, au choix et non pas arbitrairement. On prévoit en outre deux appuis : palmaire et dorsal pour cette immobilisation, afin de la rendre très efficace. L'appui palmaire constitue avantageusement un contre-appui de l'appui dorsal.

De façon intéressante, la présente invention prévoit une orthèse confortable, adaptable en fonction de l'atteinte, en position intrinsèque, et efficace.

Pour ce faire est proposé selon un premier aspect de l'invention une orthèse de main apte à immobiliser deux os métacarpiens jointifs, qui permet l'immobilisation de deux métacarpiens jointifs choisis du 2^{ème} au 5^{ème}, de façon amovible et ajustable et qui comprend au moins un appui rigide palmaire et un appui rigide dorsal, chacun amovible et déplaçable, notamment en fonction des os métacarpiens à immobiliser. Ainsi, on crée un appui en face dorsale de la main et un contre appui en face palmaire de la main, permettant de mettre la main en position intrinsèque, tout en maintenant les doigts en syndactylie, et adaptable selon l'atteinte du métacarpien.

Cette solution est donc parfaitement adaptée à différentes immobilisations : entre les 2^{ème} et 3^{ème} métacarpiens ; ou entre les 3^{ème} et 4^{ème} métacarpiens ; ou encore entre les 4^{ème} et 5^{ème} métacarpiens. Comme il sera explicité ci-après, l'homme de métier choisit et adapte facilement l'orthèse, selon le cas à traiter.

De façon particulière, l'orthèse comprend : un corps flexible présentant une face dite intérieure en contact avec la peau de la main et une face opposée dite extérieure à surface agrippante ; ledit corps flexible comprend une première zone apte à recouvrir la surface palmaire de la main et une deuxième zone apte à recouvrir la surface dorsale de la main ; - l'appui rigide dorsal est fixé sur la face agrippante de la deuxième zone et comprend une lame rigide courbée au niveau de son extrémité qui dépasse du corps flexible ; - l'appui rigide palmaire comprend au moins une lame courbée en extrémité, ladite lame dépassant du corps flexible dans le prolongement du métacarpien atteint.

Par surface agrippante on entend une surface apte à retenir une autre surface ; par exemple une surface de type Velcro© ou une surface adhésive ou toute autre surface apte à réaliser une fonction de fixation amovible c'est-à-dire non définitive. Une fixation amovible permet de décoller puis de fixer à nouveau une autre surface.

La première zone peut recouvrir tout ou partie de la surface palmaire de la main, de même que la deuxième zone peut recouvrir tout ou partie de la surface dorsale de la main. Par ailleurs le fait que l'appui rigide dorsal soit fixé sur une surface agrippante, permet de le déplacer facilement afin de l'adapter à une autre configuration et/ou à la taille de la main.

Selon une autre caractéristique de l'invention, ledit appui rigide palmaire est tel que la lame est insérée dans une poche prévue dans la première zone du corps flexible.

Par ailleurs, l'orthèse comprend au moins deux poches pour la ou les lames courbées de l'appui rigide palmaire. L'homme de métier peut ainsi enfiler une lame dans l'une ou l'autre des poches afin d'adapter la position de la lame au niveau de la phalange à immobiliser.

En outre, l'orthèse selon l'invention peut comprendre un insert en silicone apte à séparer deux phalanges jointives avec un écartement donné. L'insert peut préférentiellement être fixé de façon amovible, par exemple sur la surface intérieure du corps flexible, plus particulièrement sur l'extrémité dépassante de l'appui rigide dorsal.

Selon une autre caractéristique de l'invention, l'extrémité courbée de l'appui rigide dorsal est muni d'une bande ou sangle qui enserre deux phalanges de façon amovible, lesdites phalanges étant situées dans le prolongement des os métacarpiens atteints. La bande participe ainsi à l'immobilisation des métacarpes. Ses deux extrémités peuvent avantageusement se placer l'une sur l'autre pour leur fixation réciproque. Bien entendu, une matière de type Velcro© réalise cette fonction mais d'autres solutions peuvent être envisagées sans sortir du cadre de l'invention. La bande ou sangle placée autour des doigts permet de finaliser la syndactylie, trois autres bandes ou sangles permettent par ailleurs d'immobiliser la main en position intrinsèque. En outre la sangle, en combinaison avec l'appui dorsal, relié à l'appui palmaire permet de créer la syndactylie et d'empêcher tous mouvements des doigts et du métacarpien.

De façon intéressante, ledit corps flexible comprend au moins une bande de fermeture pour l'ajustage et le serrage autour de la main et/ou du poignet.

Plus précisément, la bande de fermeture s'étend sensiblement perpendiculairement à la longueur de la main, coopère avec un anneau pour son repli et est munie à son extrémité libre d'une surface agripante apte à se fixer de façon amovible sur la surface extérieure du corps flexible.

Tout système de fermeture amovible du corps flexible peut être prévu sans sortir du cadre de l'invention. Il s'agit en effet de pouvoir adapter et serrer le corps flexible autour de la main de façon appropriée et aussi de pouvoir modifier une position donnée, en cas de problème par exemple en cas de gonflement de la main et/ou du poignet.

Par ailleurs l'orthèse étant un dispositif directement au contact d'un membre blessé (ou atteint) qui peut être immobilisé pendant une longue période, elle comprend en outre une bande de mousse fixée sur la surface intérieure du corps flexible, apte à adoucir le contact avec la peau et placée au niveau des métacarpiens perpendiculairement à leur longueur.

### BREVE DESCRIPTION DES FIGURES

D'autres caractéristiques et avantages de l'invention ressortiront à la lecture de la description qui suit, en référence aux figures annexées, qui illustrent :
- la figure 1, une première vue de la mise en place de l'orthèse sur une main, côté dorsal ;
- la figure 2, une deuxième vue de la mise en place de l'orthèse sur une main, côté dorsal ;
- la figure 3, une vue de côté de la mise en place de l'orthèse sur une main ;
- la figure 4, une vue de côté de l'orthèse, quasiment en place sur une main ;
- la figure 5, une vue de l'orthèse du côté de sa face extérieure ;
- la figure 6, une vue d'une partie de l'orthèse du côté de sa face intérieure ;
- la figure 7, une vue d'un appui rigide palmaire faisant partie de l'orthèse ;
- la figure 8, une vue de l'orthèse aplatie du côté de sa face intérieure ;
- la figure 9, une vue d'un appui rigide dorsal mis à plat ; et
- la figure 10, une perspective de l'appui rigide dorsal.

Pour plus de clarté, les éléments identiques ou similaires sont repérés par des signes de référence identiques sur l'ensemble des figures.

### DESCRIPTION DETAILLEE D'UN MODE DE REALISATION

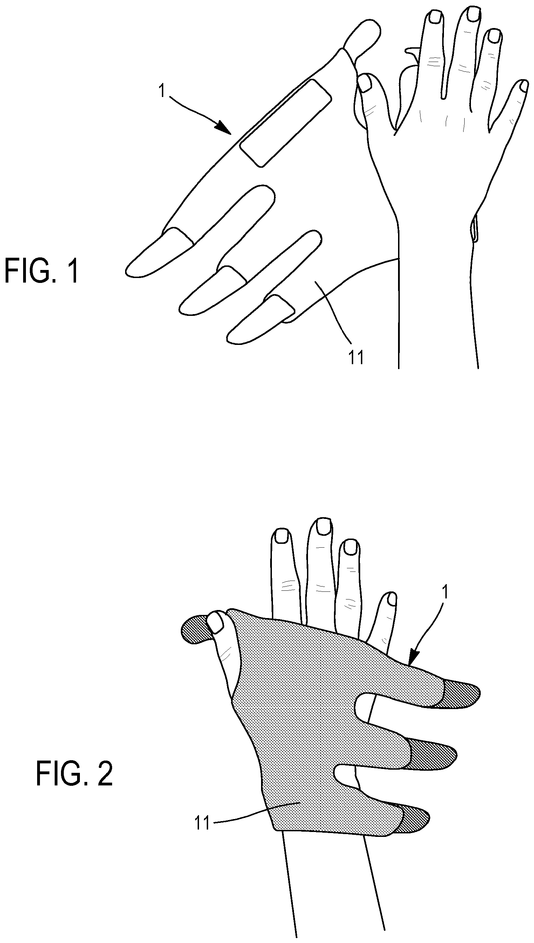

Les figures 1 à 4 illustrent la mise en place d'une orthèse selon l'invention. On voit notamment que l'orthèse recouvre le poignet et la paume de la main en laissant les doigts découverts. L'orthèse ici montrée est dédiée à la main gauche.

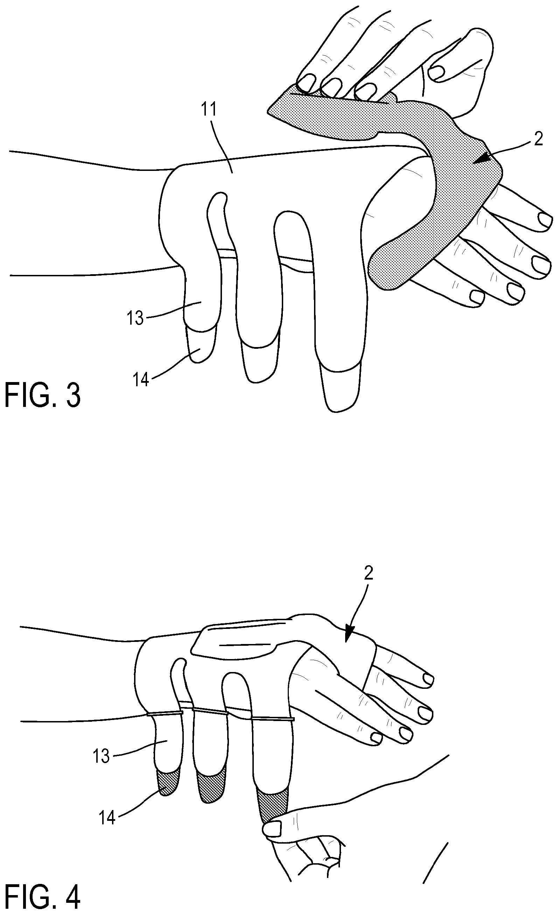

La figure 1 montre la première étape à savoir la mise en place de la paume de la main contre la face interne d'une partie de l'orthèse ; la figure 2 illustre l'étape suivante qui consiste à recouvrir la face dorsale de la main avec l'autre partie de l'orthèse ; la figure 3 montre le placement d'un appui dorsal sur la face dorsale de l'orthèse ; la figure 4 concerne la fixation des bandes de serrage autour de la main. Ces figures font ressortir la facilité de mise en place de l'orthèse.

De façon intéressante, l'organe de maintien dorsal est simplement positionné en face du métacarpien atteint puis les sangles enveloppent les doigts, la main et le poignet. La mise en place de l'orthèse est particulièrement simple et sans risque de mauvais positionnement du fait de l'aide au positionnement sur l'orthèse. La combinaison de l'organe de maintien et des sangles assure une bonne efficacité de l'orthèse sans pour autant nuire au confort de port.

Les figures suivantes vont permettre de décrire plus en détails les éléments constitutifs de l'invention.

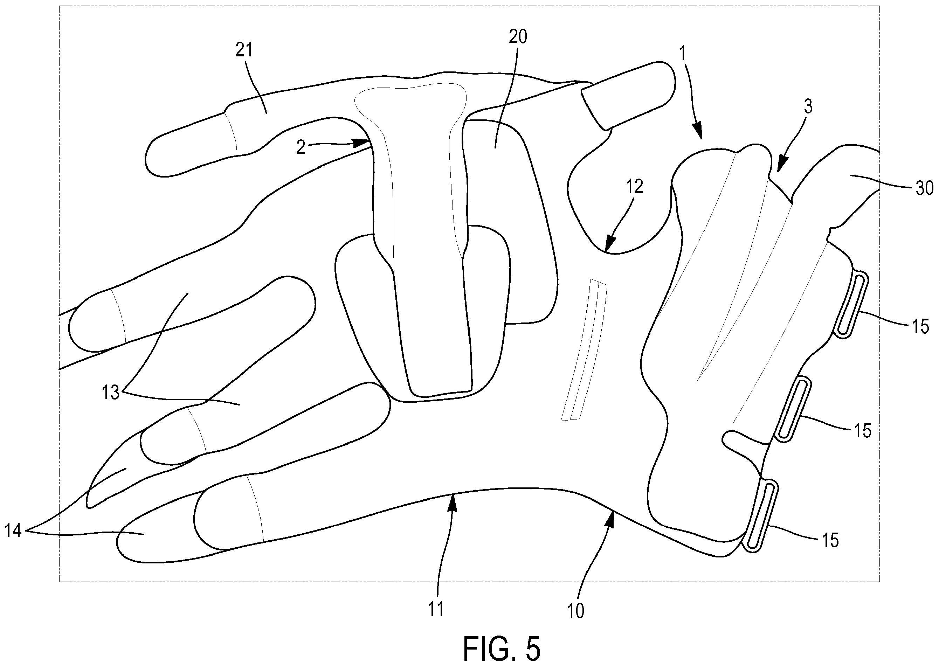

La figure 5 illustre un corps flexible 1 constitutif de l'orthèse selon l'invention, mis à plat pour les besoins de l'exposé. Plus précisément c'est la face dite extérieure du corps flexible qui est visible ; cette face est préférentiellement constituée d'une matière agrippante apte à fixer de manière amovible une autre matière agrippante complémentaire, pour les fonctions qui seront explicitées ci-après. Le corps flexible est par exemple réalisé en une matière textile, ou une mousse, flexible, légèrement élastique, d'une épaisseur de quelques millimètres.

Le corps flexible 1 comprend une première zone 10 qui recouvre tout ou partie de la surface palmaire de la main, et une deuxième zone 11 qui recouvre tout ou partie de la surface dorsale de la main. La première 10 et la deuxième 11 zones sont reliées par une zone de liaison 12 qui s'étend à proximité du métacarpe du pouce lorsque l'orthèse est placée sur la main. Un bord de forme arrondie permet de créer une ouverture pour le pouce.

Par ailleurs la deuxième zone 11 présente au moins une extension latérale 13 ou bande de fermeture, apte à refermer l'orthèse sur la main. La ou les bandes de fermeture 13 s'étendent sensiblement perpendiculairement à la longueur de la main ; la longueur de la main correspond sensiblement à l'axe général d'extension des doigts. La ou les bandes de fermeture sont ici pourvues à leurs extrémités libres d'une surface agrippante 14 par exemple constituée de boucles plastiques rigides aptes à se fixer sur une surface agrippante complémentaire. La ou les bandes 13 viennent coulisser dans des boucles ou anneaux 15 sur lesquelles elles se plient lorsque l'orthèse est en place sur la main. Chaque boucle 15 est fixée, par exemple cousue, au niveau du bord latéral de la première zone 10. Un tel système permet l'ajustage et le serrage de l'orthèse autour de la main et/ou du poignet. Bien entendu d'autres systèmes remplissant cette fonction peuvent être prévus sans sortir du cadre de l'invention.

Un appui rigide dorsal 2 est fixé de façon amovible sur la face agrippante de la deuxième zone 11 du corps flexible. La fixation amovible peut être réalisée par le contact de l'une des faces de l'appui rigide dorsal 2 avec la face agrippante de la deuxième zone 11. D'autres façons de réaliser cette fixation amovible peuvent être prévues sans sortir du cadre de l'invention. Comme on le comprendra aisément, le fait de pouvoir fixer puis déplacer l'appui rigide dorsal permet de le positionner en fonction du métacarpien atteint, ce qui est un avantage décisif vis-à-vis des systèmes connus.

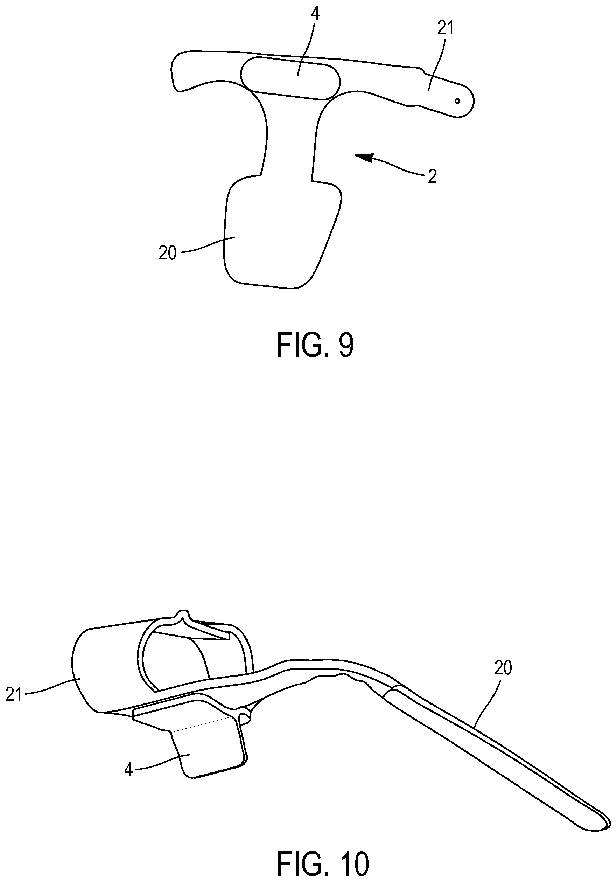

L'appui rigide dorsal 2 est représenté plus en détail sur les figures 9 et 10. Il présente ici globalement la forme d'un T dont la grande jambe 20 s'étend sensiblement selon la longueur de l'orthèse et dont la petite jambe 21 entoure les doigts comme il sera expliqué plus tard. La petite jambe 21 du T est disposée pour s'étendre au-delà du bord de la deuxième zone 11 du corps rigide. L'appui rigide 2 comprend une tige rigide par exemple en métal, aluminium ou plastique thermo-formable qui s'étend selon la grande jambe 20 du T et constitue donc la rigidité de ce composant. La tige rigide est par ailleurs courbée comme visible sur la figure 10 : sa première direction correspond au plan de la face dorsale de la main tandis que sa seconde direction est celle de l'appui 'dorsal' d'au moins une phalange. Cette pièce courbée crée l'angulation de flexion de l'articulation métacarpo-phalangienne ; l'angle d'angulation est préférentiellement d'environ 70°C. La tige rigide est préférentiellement logée dans un tissu ou autre matériau protecteur. Si la face de l'appui dorsal 2 destinée à être en contact avec la face agripante de la deuxième zone 11 n'est pas apte à agripper cette face, alors on rend cette coopération possible en ajoutant une pièce adéquate. Chacune des extrémités de la petite jambe 21 du T coopère afin de pouvoir entourer et maintenir deux doigts côte à côte.

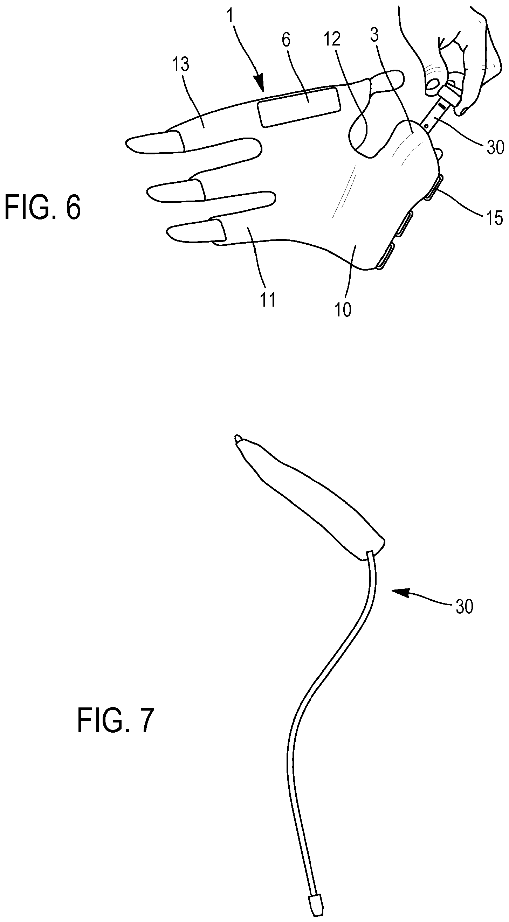

Un appui rigide palmaire 3 tel que représenté par ailleurs sur les figures 6 et 7 est prévu dans la première zone 10 du corps flexible. Il comprend une tige ou lame rigide 30, par exemple métallique, logée dans une première poche prévue dans cette première zone 10 et qui en dépasse par le bord le plus proche des doigts. La tige 30 est courbée : elle comprend une première direction qui s'étend dans le plan de la face palmaire c'est-à-dire selon le plan de la première zone 11 du corps flexible ; la seconde direction de la tige 30 est sensiblement perpendiculaire à sa première direction, de telle sorte que lorsqu'elle est en place sur la main, elle constitue un appui ' palmaire' d'au moins un doigt. La courbure de l'appui palmaire respecte la forme palmaire de la main. Vus les positionnements respectifs choisis, l'appui palmaire ' constitue un contre-appui pour 'l'appui dorsal ' formé par une partie de l'appui rigide dorsal, tel que décrit ci-avant.

La tige ou lame 30 peut être insérée dans une deuxième poche prévue dans la première zone 10. La deuxième poche présente une orientation différente de la première poche de sorte à ce que la tige 30 puisse constituer un appui pour un autre doigt. On jouera par ailleurs sur le positionnement général du corps flexible 1 vis-à-vis de la main afin de positionner l'appui palmaire au niveau du doigt correspondant au métacarpe atteint.

On comprend ainsi que les appuis palmaire et dorsal sont chacun amovible et déplaçable vis-à-vis du corps flexible 1 c'est-à-dire en fait vis-à-vis des métacarpiens à immobiliser : L'appui dorsal 2 peut se fixer de façon amovible sur la face extérieure de la seconde zone 11 tandis que l'appui palmaire 3 peut présenter plusieurs orientations relatives vis-à-vis de la première zone 10. Ces positionnements choisis permettent d'obtenir un appui et un contre-appui qui coopèrent ensemble et contribuent à une parfaite immobilisation d'un métacarpien atteint.

De plus l'orthèse selon l'invention permet une visualisation d'une partie des doigts de la main, ce qui autorise une vérification immédiate et claire de l'état de la main ; les résines ne permettent pas cette fonction.

L'orthèse selon l'invention peut présenter plusieurs longueurs afin de s'adapter à plusieurs tailles de mains notamment adulte, enfants.

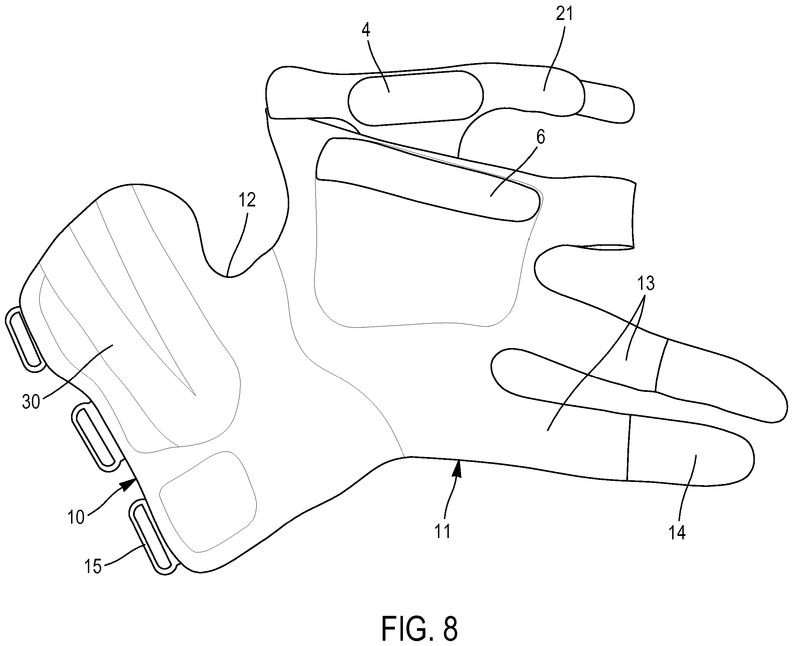

La figure 8 montre d'autres aspects intéressants de l'invention, non encore décrits : un insert 4, préférentiellement en silicone, est fixé de façon amovible au niveau de la bande 21, sur la face intérieure du corps flexible 1, en contact avec la peau des doigts de la main. L'insert 4 est apte à séparer deux phalanges juxtaposées, avec un écartement donné qui correspond sensiblement avec son épaisseur. On respecte ainsi l'espace naturel entre les doigts, sans pression particulière. Ceci constitue un avantage intéressant vis-à-vis des résines qui, souvent, serrent de façon trop forte les doigts. Vue la forme de l'insert 4 dans ce mode de réalisation de l'invention, l'écartement correspond sensiblement à deux épaisseurs de l'insert, mais cet écartement est illustratif.

La figure 8 montre en outre une bande en mousse 6 fixée sur la face intérieure du corps flexible, à proximité de la jonction des métacarpiens et des phalanges c'est-à-dire près du bord associé du corps flexible 1.

## Revendications

1. Orthèse de main apte à immobiliser deux os métacarpiens jointifs permettant l'immobilisation de deux métacarpiens jointifs choisis du 2^{ème} au 5^{ème}, de façon amovible et ajustable et comprenant des appuis rigides palmaire (3) et dorsal (2), chacun comprenant une lame rigide courbée au niveau de son extremité, ladite orthèse comprenant un corps flexible (1) présentant une face dite intérieure en contact avec la peau de la main et une face opposée dite extérieure à surface agrippante , ledit corps flexible comprenant une première zone (10) apte à recouvrir la surface palmaire de la main et une deuxième zone (11) apte à recouvrir la surface dorsale de la main et comprenant la face agrippante, les première et deuxième zones étant reliées par une zone de liaison (12) ; **caractérisée en ce que** l'appui rigide dorsal (2) est apte à être fixé sur la face agrippante de la deuxième zone (11), ladite lame rigide dépassant du corps flexible ; - ladite lame rigide de l'appui rigide palmaire (3) étant configuré pour dépasser du corps flexible dans le prolongement du métacarpien atteint

2. Orthèse selon la revendication 1 **caractérisée en ce que** ladite lame (30) dudit appui rigide palmaire (3) est insérée dans une poche prévue dans la première zone (10) du corps flexible.

3. Orthèse selon la revendication 1 ou la revendication 2 **caractérisée en ce qu'**elle comprend au moins deux poches pour la ou les lames courbées (30) de l'appui rigide palmaire.

4. Orthèse selon l'une des revendications 1 à 3 **caractérisée en ce qu'**elle comprend en outre un insert en silicone (4) apte à séparer deux phalanges jointives avec un écartement donné.

5. Orthèse selon la revendication 4 **caractérisé en ce que** l'insert (4) est fixé de façon amovible sur l'extrémité dépassante de l'appui rigide dorsal (2).

6. Orthèse selon l'une des revendications 1 à 5 **caractérisé en ce que** l'extrémité courbée de l'appui rigide dorsal (2) est muni d'une bande (21) qui enserre deux phalanges de façon amovible, lesdites phalanges étant situées dans le prolongement des os métacarpiens atteints.

7. Orthèse selon l'une des revendications 1 à 6 **caractérisée en ce que** ledit corps flexible (1) comprend au moins une bande de fermeture (13) pour l'ajustage et le serrage autour de la main et/ou du poignet.

8. Orthèse selon la revendication 7 **caractérisée en ce que** la bande de fermeture (13) s'étend sensiblement perpendiculairement à la longueur de la main, coopère avec un anneau (15) pour son repli et est munie à son extrémité libre d'une surface agripante (14) apte à se fixer de façon amovible sur la surface extérieure du corps flexible (1).

9. Orthèse selon l'une des revendications 1 à 8 **caractérisée en ce qu'**elle comprend en outre au moins une bande de mousse (6) fixée sur la surface intérieure du corps flexible, apte à adoucir le contact avec la peau et placée au niveau des métacarpiens perpendiculairement à leur longueur.

## Patentansprüche

1. Handorthese, die imstande ist, zwei aneinanderliegende Mittelhandknochen ruhigzustellen, die die lösbare und einstellbare Ruhigstellung von zwei aneinanderliegenden Mittelhandknochen, ausgewählt aus dem 2. und dem 5., erlaubt, und feste Abstützungen palmar (3) und dorsal (2) umfasst, wobei jede eine gekrümmte feste Lamelle im Bereich ihres Endes umfasst, wobei die Orthese einen flexiblen Körper (1) umfasst, aufweisend eine innere Fläche im Kontakt mit der Haut der Hand und eine gegenüberliegende äußere Fläche mit einer Klammeroberfläche, wobei der flexible Körper eine erste Zone (10) umfasst, die imstande ist, die palmare Oberfläche der Hand zu bedecken, und eine zweite Zone (11), die imstande ist, die dorsale Oberfläche der Hand zu bedecken und die Klammerfläche umfasst, wobei die erste und zweite Zone durch eine Verbindungszone (12) verbunden sind; **dadurch gekennzeichnet, dass**
- die feste dorsale Abstützung (2) imstande ist, auf der Klammerfläche der zweiten Zone (11) befestigt zu sein, wobei die feste Lamelle über den flexiblen Körper hervorsteht,
- wobei die feste Lamelle der festen palmaren Abstützung (3) ausgebildet ist, um über den flexiblen Körper in der Verlängerung des angegriffenen Mittelhandknochens hervorzustehen.

2. Orthese nach Anspruch 1, **dadurch gekennzeichnet, dass** die Lamelle (30) der festen palmaren Abstützung (3) in eine Tasche eingeführt ist, die in der ersten Zone (10) des flexiblen Körpers vorgesehen ist.

3. Orthese nach Anspruch 1 oder Anspruch 2, **dadurch gekennzeichnet, dass** sie mindestens zwei Taschen für die gekrümmte(n) Lamelle(n) (30) der festen palmaren Abstützung umfasst.

4. Orthese nach einem der Ansprüche 1 bis 3, **dadurch gekennzeichnet, dass** sie ferner einen Einsatz aus Silikon (4) umfasst, der imstande ist, zwei aneinanderliegende Fingerglieder mit einer bestimmten Beabstandung zu trennen.

5. Orthese nach Anspruch 4, **dadurch gekennzeichnet, dass** der Einsatz (4) lösbar auf dem Ende befestigt ist, das über die feste dorsale Abstützung (2) hervorsteht.

6. Orthese nach einem der Ansprüche 1 bis 5, **dadurch gekennzeichnet, dass** das gekrümmte Ende der festen dorsalen Abstützung (2) mit einem Band (21) ausgestattet ist, das zwei Fingerglieder lösbar umschließt, wobei sich die Fingerglieder in der Verlängerung der angegriffenen Mittelhandknochen befinden.

7. Orthese nach einem der Ansprüche 1 bis 6, **dadurch gekennzeichnet, dass** der flexible Körper (1) mindestens ein Verschlussband (13) zum Einstellen und Umschließen der Hand und/oder des Handgelenks umfasst.

8. Orthese nach Anspruch 7, **dadurch gekennzeichnet, dass** sich das Verschlussband (13) etwa senkrecht zur Länge der Hand erstreckt, mit einem Ring (15) für seine Faltung zusammenwirkt und an seinem freien Ende mit einer Klammeroberfläche (14) ausgestattet ist, die imstande ist, sich lösbar auf der äußeren Oberfläche des flexiblen Körpers (1) zu befestigen.

9. Orthese nach einem der Ansprüche 1 bis 8, **dadurch gekennzeichnet, dass** sie ferner mindestens ein Schaumstoffband (6) umfasst, das auf der inneren Oberfläche des flexiblen Körpers befestigt ist, das imstande ist, den Kontakt mit der Haut abzupolstern und im Bereich der Mittelhandknochen senkrecht zu deren Länge platziert ist.

## Claims

1. Hand orthosis capable of immobilising two contiguous metacarpal bones enabling the immobilisation of two contiguous metacarpals chosen from the 2^{nd} to the 5^{th}, in a removeable and adjustable manner and comprising rigid palmar (3) and dorsal (2) supports, each comprising a rigid blade curved at the end thereof, said orthosis comprising a flexible body (1) having a so-called inner face in contact with the skin of the hand and an opposite so-called outer face with a gripping surface, said flexible body comprising a first zone (10) capable of covering the palmar surface of the hand and a second zone (11) capable of covering the dorsal surface of the hand and comprising the gripping face, the first and second zones being connected by a connection zone (12); **characterised in that** - the rigid dorsal support (2) is capable of being attached on the gripping face of the second zone (11), said rigid blade protruding from the flexible body; - said rigid blade of the rigid palmar support (3) being configured to protrude from the flexible body in the continuation of the affected metacarpal.

2. Orthosis according to claim 1 **characterised in that** said blade (30) of said rigid palmar support (3) is inserted into a pocket provided in the first zone (10) of the flexible body.

3. Orthosis according to claim 1 or claim 2 **characterised in that** it comprises at least two pockets for the curved blade or blades (30) of the rigid palmar support.

4. Orthosis according to one of claims 1 to 3 **characterised in that** it further comprises a silicone insert (4) capable of separating two contiguous phalanges with a given spacing.

5. Orthosis according to claim 4 **characterised in that** the insert (4) is attached in a removable manner on the protruding end of the rigid dorsal support (2).

6. Orthosis according to one of claims 1 to 5 **characterised in that** the curved end of the rigid dorsal support (2) is equipped with a strip (21) that encloses two phalanges in a removable manner, said phalanges being located in the continuation of the affected metacarpal bones.

7. Orthosis according to one of claims 1 to 6 **characterised in that** said flexible body (1) comprises at least one closure strip (13) for the adjustment and the tightening around the hand and/or the wrist.

8. Orthosis according to claim 7 **characterised in that** the closure strip (13) extends substantially perpendicular to the length of the hand, cooperates with a ring (15) for the folding thereof and is equipped at the free end thereof with a gripping surface (14) capable of attaching in a removable manner on the outer surface of the flexible body (1) .

9. Orthosis according to one of claims 1 to 8 **characterised in that** it further comprises at least one foam strip (6) attached on the inner surface of the flexible body, capable of softening the contact with the skin and placed at the metacarpals perpendicular to the length thereof.
